(19) Europäisches Patentamt
European Patent Office
Office européen des brevets

(11) EP 0 366 041 B2

(12) NEW EUROPEAN PATENT SPECIFICATION

(45) Date of publication and mention of the opposition decision:
**12.09.2001 Bulletin 2001/37**

(45) Mention of the grant of the patent:
**05.08.1998 Bulletin 1998/32**

(21) Application number: **89119644.6**

(22) Date of filing: **23.10.1989**

(51) Int Cl.[7]: **C07D 207/408**, C07D 207/452, C07D 209/48, C11D 3/395, C11D 3/39, D06L 3/02

# (54) Imido-percarboxylic acids

Imido-percarbonsäuren

Acides imido-percarboxyliques

(84) Designated Contracting States:
**AT BE CH DE ES FR GB LI NL SE**

(30) Priority: **24.10.1988 IT 2240288**

(43) Date of publication of application:
**02.05.1990 Bulletin 1990/18**

(73) Proprietor: **AUSIMONT S.p.A.**
**I-20100 Milano (IT)**

(72) Inventors:
- **Venturello, Carlo, Dr.**
**I-28100 Novara (IT)**
- **Cavallotti, Claudio**
**I-20146 Milan (IT)**
- **Bencini, Elena, Dr.**
**I-46100 Mantova (IT)**
- **Sasso, Maria Antonietta, Dr.**
**I-10023 Chieri, Torino (IT)**

(74) Representative:
**Weinhold, Peter, Dr.rer.nat. Dipl.-Chem. et al**
**Winter, Brandl, Fürniss, Hübner,**
**Röss, Kaiser, Polte**
**Partnerschaft**
**Patent- und Rechtsanwaltskanzlei**
**Alois-Steinecker-Strasse 22**
**85354 Freising (DE)**

(56) References cited:
**EP-A- 325 288**
**EP-A- 0 349 940**
**WO-A-90/07501**
**DE-A- 1 793 378**
**DE-A- 3 823 172**
**DE-A- 3 906 768**



Printed by Jouve, 75001 PARIS (FR)

## Description

The present invention relates to percarboxylic acids which may be referred to as imido-percarboxylic acids and to a process for their preparation.

More particularly, the present invention relates to imido-percarboxylic acids having the general formula:

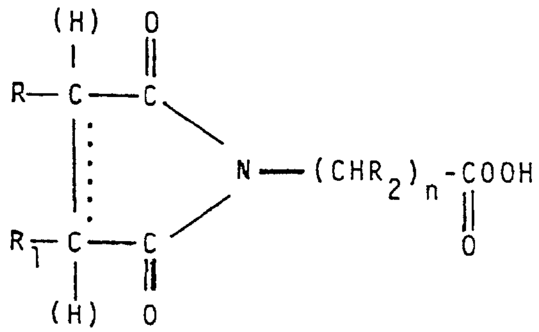

(I)

wherein:

- R and $R_1$, the same or different from each other, represent hydrogen atoms or linear or branched $C_{1-12}$-alkyl groups or R and $R_1$, together with the carbon atoms to which they are linked, form a cycloaliphatic ring containing from 4 to 12 carbon atoms; the symbol ..... indicates a single or olefinically unsaturated bond;
- $R_2$ represents a hydrogen atom, a linear or branched $C_{1-5}$-alkyi group or an OH group; and n is an integer of from 1 to 10; the groups represented by R, $R_1$ and $(CHR_2)_n$ being optionally substituted by one or more substituents selected from F, Cl, OH, $NO_2$ and $C_1$-$C_5$ alkoxy groups, which substituents may be the same or different from each other, excluding the compounds as disclaimed in claim 1.

The compounds of the above formula (I) are new compounds which constitute a class of products which are highly interesting from an industrial point of view.

In fact, similarly to the already known peracids, they may find use in general in the field of plastics, as polymerization initiators or as oxidants for olefin epoxidation, and in many other oxidative processes in the field of fine chemicals.

DE-OS 1793378 discloses N-acyl-α-amino percarboxylic add alkyl esters which are used as initiators for polymerization reactions in aqueous emulsions.

Specifically, the compounds of the above formula (I) find a particularly effective application in the field of bleaching in the detergent industry.

In the past few years organic peracids have attracted an increasing interest in industry, due, especially, to their excellent employability as bleaching agents in formulations for medium-low temperature washing and, even more important, also due to energy-saving considerations.

A large number of organic peracids has been described, endowed with the required properties of high bleaching activity and in particular, of thermal stability and storage stability or shelf life, the latter properties, of course, being essential for operations on an industrial scale and for a widespread application of compounds of this type.

Therefore, many (cyclo)aliphatic and aromatic mono- or di-percarboxylic acids are already known and used, among others, in the field of detergents.

Previously described percarboxylic acids are, e.g.; diperdodecanedioic acid, monoperoxyphthalic acid, diperazelaic acid, and substituted diperglutaric and adipic acids, etc.

Therefore, one object of the present invention is to provide novel percarboxylic acids particularly suitable for use as bleaching agents, e.g. in detergent formulations, especially those intended for low-medium temperature use.

Another object of the present invention is the provision of a facile and inexpensive process for the preparation of such compounds.

These, and still other objects which will become even more apparent for those skilled in the art from the following detailed disclosure, are achieved, according to the present invention, by the imido-percarboxylic acids of the above general formula (I), and by the process for their preparation, wherein a substrate, i.e. an imido-carboxylic acid having a structure corresponding to the desired percarboxylic acid of formula (I), is reacted with concentrated $H_2O_2$, operating in an acid reaction medium selected from concentrated $H_2SO_4$ and $CH_3SO_3H$, the resulting percarboxylic acid being subsequently separated from the reaction mixture by known methods, whereafter it is washed and dried.

With reference to the above formula (I), R and $R_1$, equal to or different from each other, represent hydrogen atoms or linear or branchend alkyl groups containing from 1 to 12, particularly from 1 to 6, carbon atoms (e.g. methyl, ethyl, n-and i-propyl, n-, sek-, i- or tert-butyl, pentyl and hexyl); moreoever, R and $R_1$, taken together with the carbon atoms to which they are linked, may give rise to a cydoaliphatic ring containing from 4 to 12 carbon atoms, particularly 5 or 6 carbon atoms.

$R_2$ represents a hydrogen atom, an OH group or a straight or branched alkyl group containing from 1 to 5, particularly from 1 to 3 carbon atoms (e.g. methyl, ethyl and n-and i-propyl).

Preferably, $R_2$ is a hydrogen atom.

Finally, n represents an integer ranging from 1 to 10, and particularly from 1 to 5. In the most preferred embodiments n is 1, 2 or 3.

Moreover, the groups R, $R_1$ and $(CHR_2)_n$ may in turn be substituted with one or more (e.g. 2 to 4) groups, either equal to or different from each other, which are inert under the reaction conditions under which the preparation takes place and are selected from $C_1$-$C_5$-alkoxy groups (e.g. methoxy and ethoxy), OH, $NO_2$, F and Cl.

Particularly preferred peracids of the above formula (I), which may be obtained according to the present invention, are 4-succinimido perbutyric acid, 3-succinimido perpropionic acid, succinimido peracetic acid, 4-cis-hexahydro-phthalimido perbutyric acid, 3-cis-hexahydro-phthalimido perpropionic acid, 2-cis-hexahydro-phthalimido-3-methyl perbutyric acid, 2-cis-hexahydro-phthalimido perpropionic acid and 4-(3,4,5-6-tetrahydro-phthalimido)perbutyric add.

The substrates used as starting materials are <u>per se</u> known compounds and/or can be prepared according to conventional techniques.

Suitable starting substrates for obtaining the corresponding imido-percarboxylic acids of formula (I) are, e.g., 4-cis-succinimido-butyric acid, 3-ds-succinimido-propionic acid, succinimido acetic acid, 4-cis-hexahydro-phthalimidobutyric acid, 3-cis-hexahydro-phthalimido propionic acid, 2-ds-hexahydrophthalimido-3-methyl butyric acid, 2-cis-hexahydro-phthalimido propionic acid and 4-(3,4,5,6-tetrahydrophthalimido)butyric acid.

According to a preferred mode of operation the percarboxylation reaction of the imido carboxylic acids used as starting substrates is carried out by gradually adding, under stirring, $H_2O_2$ having a concentration of from about 70% to about 90% by weight to a solution of the substrate in concentrated $H_2SO_4$ or $CH_3SO_3H$ and by maintaining the reaction temperature throughout the reaction within the range of from 0° to about 15°C.

Preferably, the amount of $H_2SO_4$ or $CH_3SO_3H$ employed, determined at a concentration of 100%, is at least equal to about 3 moles, particularly of from about 3 to about 20 moles, per mole of substrate.

Generally, the hydrogen peroxide is used in an amount which is in excess with respect to the substrate, and preferably is at least equal to 1.2, particularly from about 1.5 to about 6 moles, per mole of substrate.

The reaction time depends on the nature of the substrate, on the operating temperature, and on the total molar ratio $H_2SO_4/H_2O$ or $CH_3SO_3H/H_2O$ present at the end of the reaction. Preferably, said ratio is at least equal to 1, particularly from about 1.2 to about 5, which can be achieved by adjusting the various parameters.

The reation time usually is about 2 hours.

The separation of the imido-peracid of formula (I) may be carried out according to conventional methods, such as by filtration of the solid precipitate or by solvent extraction etc.

The compounds of formula (I) usually are solid at room temperature.

According to the present invention, they may be used to particular advantage in formulations of detergent compositions, e.g. granular formulations, or as bleaching agents in solution for use over a wide temperature range, e.g. of from 20°C to 90°C.

Therefore, the imido-percarboxylic acids of the present invention may be used as bleaching agents as such, i.e. not incorporated in a detergent composition, or, preferably, together with and contained in conventional detergent compositions including other components and/or additives, such as, for example, builders, surfactants, soaps, zeolites, hydrotropic agents, corrosion inhibitors, enzymes, optical bleaching agents, stabilizers and other bleaching compounds.

Preferably, the operating temperature ranges from room temperature to about 65°C.

The preparation and use of the compositions as well as their formulations are known and/or conventional.

The peracids of the present invention may be used in combination with solid and/or liquid detergent compositions, and/or in the presence of other bleaching peroxy compounds.

Moreover, the compounds of formula (I) may be subjected to known phlegmatization processes.

The present invention is disclosed in still further detail in the following examples which are provided for purely illustrative purposes without being a limitation of possible embodiments of the invention.

The products prepared in the examples were characterized by elemental analysis, by determining their content of active oxygen (by iodometric titration) and by using Fourier Transform Infrared Spectroscopy (FT-IR).

EXAMPLE 1

20 g (0.196 mole) of $H_2SO_4$ (96%) were placed into a beaker equipped with stirrer, thermometer and external bath. 4.9 g (0.1009 mole) of $H_2O_2$ (70%) and 8.5 g (0.0459 mole) of 4-succinimido-butyric acid were slowly added under stirring by maintaining the internal temperature between 0° and +5°C.
The reaction was continued for 2 hours at +15°C.
At the end, the reaction mixture was poured into 100 ml of a 40% aqueous solution of $(NH_4)_2SO_4$ maintained under stirring at +5°C.
The resulting mixture was extracted with $CH_2Cl_2$ (6 x 50 ml). The combined extracts were then washed with 30 ml of aqueous $(NH_4)_2SO_4$ (40%) and finally were dried over anhydrous $Na_2SO_4$, filtered and concentrated. 7.3 g of practically pure crystalline 4-succinimido-perbutyric acid were obtained. Yield: 80%.

| Elemental Analysis: | | | | |
|---|---|---|---|---|
| Calculated for $C_8H_{11}O_5N$ | C: 47.76%; | H: 5.51%, | N: 6.96%; | O (active): 7.95% |
| Found | C: 48.2%; | H:5.58%; | N:6.89%; | 0 (active): 7.93% |

Melting point: 92°C (with decomposition).

EXAMPLE 2

The procedure of example 1 was followed, using 7.3 g (0.0715 mole) of sulphuric acid at 96%, 1.5 g (0.0375 mole) of $H_2O_2$ at 85%, and 3.3 g (0.0193 mole) of 3-succinimido propionic acid. After 2 hours of stirring at +15°C the reaction product was poured into 70 ml of 40% aqueous $(NH_4)_2SO_4$ maintained under stirring at +5°C. The resulting mixture was then extracted with ethyl acetate (8 x 40 ml) and the procedure of example 1 was followed. After solvent evaporation, an oily material was obtained which was dissolved in ethyl acetate and precipitated as a solid with petroleum ether, maintaining the mixture under stirring until solidification was complete. 3 g of substantially pure 3-succinimido perpropionic acid were separated by filtration. Yield: 83%.

| Elemental Analysis: | | | | |
|---|---|---|---|---|
| Calculated for $C_7H_9NO_5$ | C: 44.92%; | H: 5.84%; | N: 7.48%; | 0 (active): 8.55%. |
| Found | C: 45.1%; | H: 4.98%; | N: 7.45%; | 0 (active): 8.50%. |

Melting point: 78°C (with decomposition).

EXAMPLE 3

The procedure of example 1 was followed, using 5.2 g (0.0509 mole) of $H_2SO_4$ at 96%, 1.5 g (0.0375 mole) of $H_2O_2$ at 85% and 2 g (0.0127 mole) of succinimidoacetic acid. After 2 hours of stirring at +15°C, dilution with 30 ml of 40% aqueous $(NH_4)_2SO_4$, extraction with ethyl acetate (4 x 30 ml) and evaporation of the solvent, a solid product was separated which was recrystallized by dissolving it in ethyl acetate and reprecipitating it with petroleum ether. 1.3 g of crystalline substantially pure succinimido peracetic acid were obtained. Yield: 60%.

| Elemental Analysis: | | | | |
|---|---|---|---|---|
| Calculated for $C_6H_7NO_5$ | C: 41.62%; | H: 4.07%; | N: 8.09%; | 0 (active): 9.24%. |
| Found | C: 41.95%; | H: 4.10%; | N: 8.06%; | 0 (active) : 9.23%. |

The product started to decompose at 83°C and was completely molten at 107°C.

EXAMPLE 4

The procedure of example 1 was followed, using 3.6 g (0.0352 mole) of $H_2SO_4$ at 96%, 0.8 g (0.02 mole) of $H_2O_2$ at 85% and 1.7 g of 4-cis-hexahydro-phthalimido butyric acid. After 2 hours of stirring at +15°C, dilution at +5°C with 30 ml of aqueous $(NH_4)_2SO_4$ (40%), extraction with ethyl acetate (3 x 40 ml) and evaporation of the solvent, 1.6 g of an oily material were separated. Said material could not be crystallized and had an active oxygen content of 6.2% (the the orical content of 4-cis-hexahydro-phthalimido perbutyric acid is 6.26%).

Yield: 88%.

| Elemental Analysis: | | | | |
|---|---|---|---|---|
| Calculated for $C_{12}H_{17}NO_5$<br>Found | C: 56.46%;<br>C: 56.76%; | H: 6.71%;<br>H: 6.92%; | N: 5.48%;<br>N: 5.41%; | 0 (active): 6.26%.<br>0 (active): 6.20%. |

The product decomposed at 32°C.

EXAMPLE 5

By operating according to the process conditions of example 1, 18 g (0.176 mole) of $H_2SO_4$ at 96%, 4 g (0.1 mole) of $H_2O_2$ at 85% and 8 g of 3-cis-hexahydro-phthalimido propionic acid were used.
After 2 hours of stirring at +15°C, subsequent dilution at +5°C with 100 ml of aqueous $(NH_4)_2SO_4$ (40%), successive extraction with ethyl ether (3 x 50 ml) and evaporation of the solvent, a solid product was separated which was purified by dissolution in ethyl ether and reprecipitation with petroleum ether. 7 g of crystalline, substantially pure cis-hexahydro-phthalimido perpropionic acid were obtained.

Yield: 82%.

| Elemental Analysis: | | | | |
|---|---|---|---|---|
| Calculated for $C_{11}H_{15}NO_5$<br>Found | C: 54.76%;<br>C: 55.2%; | H: 6.26%;<br>H: 6.37%; | N: 5.80%;<br>N: 5.73%; | 0 (active): 6.63%.<br>0 (active): 6.60%. |

Melting point: 55°C (with decomposition).

EXAMPLE 6

By operating according to the process conditions of example 1, 23.6 g (0.231 mole) of $H_2SO_4$ at 96%, 6.8 g (0.14 mole) of $H_2O_2$ at 70% and 11.8 g (0.0466 mole) of 2-cis-hexahydro-phthalimido-3-methylbutyric acid were used.
After 2 hours of stirring at +15°C, dilution with 100 ml of aqueous $(NH_4)_2SO_4$ (40%), at a temperature of +5°C, extraction with ethyl ether (3 x 60 ml) and evaporation of the solvent, an oily material was separated which was dissolved in ethyl ether and precipitated as a solid with petroleum ether. 7.8 g of 2-cis- hexahydro-phthalimido-3-methyl perbutyric acid having a purity of 98.7% were obtained.

Yield: 61%.

| Elemental Analysis: | | | | |
|---|---|---|---|---|
| Calculated for $C_{13}H_{19}NO_5$<br>Found | C: 57.98%;<br>C: 57.74%; | H: 7.11%;<br>H: 7.14%; | N:5.20%;<br>N: 5.17%; | 0 (active): 5.94%.<br>0 (active): 5.86%. |

Melting point: 92°C (with decomposition).

EXAMPLE 7

The procedure of example 1 was followed, using 1.6 g (0.04 mole) of $H_2O_2$ at 85% and 3 g (0.0133 mole) of 2-cis-hexahydrophthalimido propionic acid. After 2 hours of stirring at +15°C, subsequent dilution with 40 ml of 40% aqueous $(NH_4)_2SO_4$ maintained under stirring at +5°C, subsequent extraction with ethyl ether (3 x 40 ml) and evaporation of the solvent, an oily product was separated which was dissolved in $CH_2Cl_2$ and precipitated as a solid with petroleum ether. The product was crystallized from ethyl acetate/petroleum ether to obtain 1.8 g of crystalline, 98%

pure 2-cis-hexahydrophthalimido perpropionic acid.

Yield: 55%.

| Elemental Analysis: | | | | |
|---|---|---|---|---|
| Calculated for $C_{11}H_{15}NO_5$ | C: 54.76%; | H: 6.26%; | N: 5.80%; | 0 (active): 6.63%. |
| Found | C: 54.51%; | H: 6.53%; | N: 5.79%; | 0 (active): 6.49%. |

Melting point: 77°C (with decomposition).

<u>EXAMPLE 8</u>

2 g (0.0084 mole) of 4-(3,4,5,6-tetrahydrophthalimido)butyric acid were dissolved, at room temperature, in 5.2 g of methanesulphonic acid. 0.7 g (0.0175 mole) of $H_2O_2$ at 85% were added to the solution which was maintained under stirring at +10°C in a way such that the temperature did not exceed +15°C.
The reaction was continued for 2 hours at +15°C.
The reaction product was poured into 20 ml of 40% aqueous $(NH_4)_2SO_4$, maintained under stirring at +5°C, and the resulting mixture was extracted with ethyl ether (2 x 30 ml). The ether extract was washed with 20 ml of 40% aqueous $(NH_4)_2SO_4$, dried over anhydrous $Na_2SO_4$, filtered and concentrated.
A viscous oily residue was obtained which, redissolved in ethyl ether, was precipitated as a solid with petroleum ether. 1.5 g of substantially pure 4-(3,4,5,6-tetrahydrophthalimido) perbutyric acid were separated by filtration.

Yield: 70%.

| Elemental Analysis: | | | | |
|---|---|---|---|---|
| Calculated for $C_{12}H_{15}NO_5$ | C: 56.91%; | H: 5.97%; | N: 5.53%; | 0 (active): 6.32%. |
| Found | C: 56.65%; | H: 5.90%; | N: 5.47%; | 0 (active): 6.31%. |

The melting temperature of the product was between 42° and 54°C with decomposition.

<u>EXAMPLE 9</u> (Application example)

<u>Bleaching with 4-succinimido perbutyric acid</u>

Bleaching tests were carried out with a detergent formulation containing 4-succinimidoperbutyric acid in the amount reported in the following Table 1 (composition A) and a similar comparative formulation containing, as bleaching agent, H 48 peracid (Mg salt of monoperphthalic add, a known commercial product, manufactured by INTEROX Chemical Ltd London, U.K., for use in the detergent art) (composition B).
Compositions A and B were obtained by dry blending of a detergent base (identical for both compositions) with the above bleaching agents. As detergent base a granular composition was used which contained all of the conventional components of a detergent for washing machines (surfactants, builders, etc.), except the chemical bleaching agents, and was obtained by atomization of the component mixture. The detergent base employed had the following composition:

| | Weight % |
|---|---|
| Total surfactants | 15.4 |
| Sodium alkyl ($C_{12}$) benzenesulphonate, soap, ethoxylated (EO) alcohol ($C_{16}$-$C_{18}$) Total sodium phosphates | 8.8 |
| Zeolite A | 19.8 |
| Silicate ($SiO_2/Na_2O$=2) | 4.4 |
| Sodium sulphate | 36.6 |

(continued)

| | Weight % |
|---|---|
| Sodium carbonate | 6.6 |
| Carboxymethylcellulose | 1.1 |
| Anti-encrusting copolymers | 4.8 |
| Water | 2.2 |
| Optical bleaching agents | 0.3 |

The metering of the compositions A and B was carried out in a way such as to introduce int> the washing machine a constant amount of detergent base (corresponding to 120 g) and an amount of bleaching agent such as to introduce into the washing machine a quantity of total initial active oxygen of about 2g of oxygen for each washing cycle, identical for all operations.

Specifically, the compositions A and B given in the following Table 1 were used in the bleaching tests.

TABLE 1

| Composition A | |
|---|---|
| Detergent base | 120 g |
| 4-Succinimido-perbutyric acid having 7.7% of active oxygen | 26 g |
| **Composition B** | |
| Detergent base | 120 g |
| H 48 having 5.5% of active oxygen | 39 g |

The tests were carried out in a commercial IGNIS® Mod. 644 washing machine by introducing into the machine two cotton specimens (15 x 15 cm) stained with standard stains or red wine at the EMPA INSTITUTE of St. Gallen (Switzerland) and marked with the "EMPA 114" mark, together with 3 kg of cleaned cotton wipers as ballast for each washing operation.
The washing operations were carried out by using a conventional low temperature program (about 40°C). Normal tap water, having a hardness of 14°F, was used.
The results of the tests are reported in the following Table 2, wherein the data are expressed as % Bleaching, defined as:

$$\% \text{ Bleaching} = \frac{A - B}{C - B} \times 100$$

wherein:

A = degree of whiteness (%) of the specimen bleached after the test;
B = degree of whiteness (%) of the specimen before the test;
C = degree of whiteness (%) of the completely bleached specimen.

The degree of whiteness was measured by means of an Elrepho Zeiss Reflectometer, using a filter No. 6 ($\lambda$ = 464 nm) and assuming MgO = 100% of whiteness.

TABLE 2

| | % Bleaching |
|---|---|
| Composition A | 58 |
| Composition B | 52 |

The data show the higher bleaching power of the claimed peracid in comparison to that of H 48.

<u>EXAMPLE 10</u> (Application example)

Bleaching tests were carried out with the imido-peracids listed in the following Tables 3 and 4, at alkaline pH (Table 3) and acid pH (Table 4), in comparison to H 48.

All tests were carried out at constant temperature of 40°C and 60°C with an initial concentration of total active oxygen in the bleaching solution of 200 mg/l, identical for all products.

Process:

For each test, 500 ml of deionized water, contained in a 1,000 ml flask equipped with a condenser, were heated to a temperature of 40°C or 60°C and adjusted to a pH of 9.5 (with NaOH) (Table 3) or to a pH of 3-4 (corresponding to the pH value which may be obtained by the direct dissolution of the present peracids) (Table 4). Then the bleaching product was added, under stirring, in the amounts given in the following tables and immediately thereafter two cotton specimens (10 cm x 10 cm) stained with standard stains of red wine at EMPA INSTITUTE of St. Gallen (Switzerland), and marked with the "EMPA 114" mark, were added.

Thereafter, the system was stirred for 60 minutes and finally the specimens, rinsed under running water, dried and ironed, were subjected to an evaluation of the bleaching effect by determining the degree of whiteness by reflectometry. The obtained results are reported in the following Tables 3 and 4, expressed in terms of % Bleaching as defined in the above Example 9.

The data in Table 3 (tests carried out at alkaline pH at 60°C) show that the bleaching power of the peracids of the present invention is equal to or higher than that of H 48 and that it is much higher than that of H 48 when the washing is carried out at low temperatures (40°C).

Likewise, the data in Table 4 (which refer to tests carried out at 60°C) show that the peracids of the present invention have a particularly high bleaching power in acid solution (this is particularly surprising in consideration of the fact that peroxy compounds generally show a very modest bleaching activity under these conditions), much higher than that of H 48.

TABLE 3

| Tests Carried out at Alkaline pH (9.5) | | | | |
|---|---|---|---|---|
| Compound | Amount used in the test (grams) | Initial concentration of total active oxygen (mg/l) | % Bleaching | |
| | | | at 40°C | at 60°C |
| 4-succinimido-perbutyric acid (Ex. 1) (Active oxygen: 7.9%) | 1.27 | 200 | 74 | 82 |
| 3-succinimido-perpropionic acid (Ex. 2) (Active oxygen: 8.5%) | 1.22 | 200 | 76 | 80 |
| 2-cis-hexahydrophthalimido-3-methyl-perbutyric acid (Ex. 6) (Active oxygen: 5.8%) | 1.73 | 200 | ... | 81 |
| H 48 (Active oxygen: 5.5%) | 1.86 | 200 | 67 | 80 |

TABLE 4

| Tests Carried out at Acid pH (3-4) | | | |
|---|---|---|---|
| Compound | Amount used in the test (grams) | Initial concentration of total active oxygen (mg/l) | % Bleaching |
| Example 1 (Active oxygen titre = 7.9%) | 1.27 | 200 | 76 |
| Example 2 (Active oxygen titre = 8.5%) | 1.22 | 200 | 76 |
| Example 6 (Active oxygen titre = 5.8%) | 1.73 | 200 | 79 |
| H 48 (Active oxygen titre = 5.5%) | 1.86 | 200 | 60 |

**Claims**

**1.** Imido-percarboxylic acids having the general formula (I):

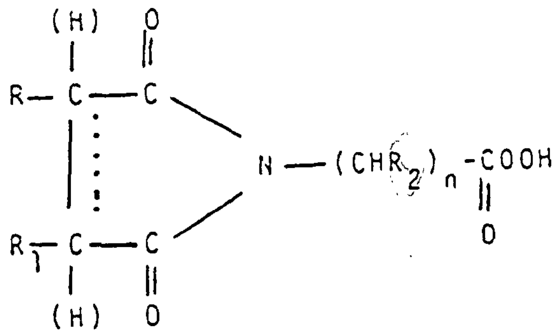

(I)

wherein:

R and $R_1$. the same or different from each other, represent hydrogen atoms or linear or branched $C_{1-12}$-alkyl groups or R and $R_1$, together with the carbon atoms to which they are linked, form a cycloaliphatic ring containing from 4 to 12 carbon atoms; the symbol ..... indicates a single or olefinically unsaturated bond;

$R_2$ represents a hydrogen atom, a linear or branched $C_{1-5}$-alkyl group or an OH group; and n is an integer of from 1 to 10; the groups represented by R, $R_1$ and $(CHR_2)_n$ being optionally substituted by one or more substituents selected from F, Cl. OH, $NO_2$ and $C_1$-$C_5$ alkoxy groups, which substituents may be the same or different from each other,

excluding the compounds defined by the general formulas a), b) and c):

a)

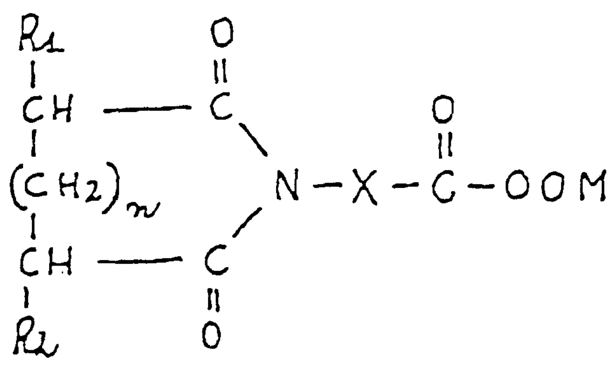

wherein:

n = zero
$R_1$ = H, $C_1$-$C_{12}$-alkyl
$R_2$ = H
M = H
X = $C_1$-$C_{10}$-alkylene

b)

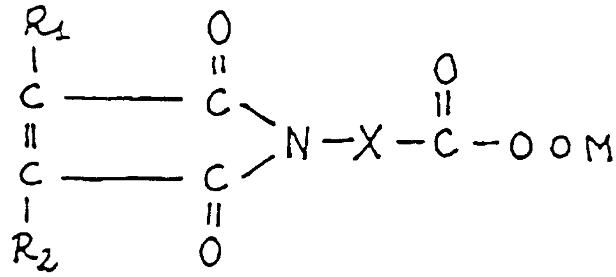

wherein:

$R_1$ = H, C1-C12-alkyl
$R_2$ = H
M = H
X = $C_1$-$C_{12}$-alkylene

c)

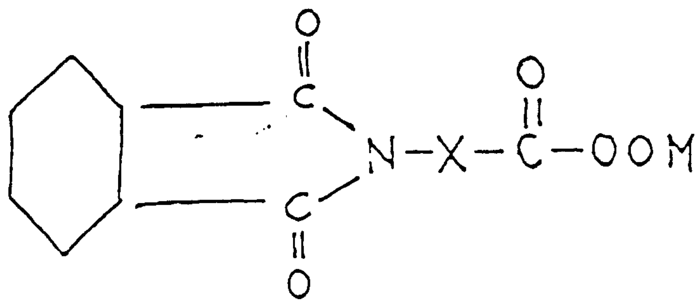

wherein:

M = H
X = $C_1$-$C_{10}$-alkylene.

**2.** Compounds according to claim 1, wherein R and $R_1$ represent linear or branched $C_{1-6}$-alkyl groups or R and $R_1$, together with the carbon atoms to which they are linked, form a cycloaliphatic ring containing 5 or 6 carbon atoms, which groups represented by R and $R_1$ may optionally be substituted as indicated in claim 1.

**3.** Compound according to claim 1, 2-hexahydrophthalimido-3-methyl-perbutyric acid, 2-hexahydro-phthalimidoperpropionic acid or 4-(3,4,5,6-tetrahydro-phthalimido) perbutyric acid.

**4.** Process for preparing the compounds according to claim 1, wherein a substrate, the imido-carboxylic acid corresponding to the desired imido-percarboxylic acid of formula (I), is reacted with concentrated $H_2O_2$ by operating in a medium selected from concentrated $H_2SO_4$ and $CH_3SO_3H$, the resulting compound of formula (I) being separated thereafter from the reaction mixture by known techniques.

**5.** Process according to claim 4, wherein the substrate is selected from 2-hexahydro-phthalimido-3-methyl-butyric acid, 2-hexahydrophthalimido propionic acid and 4-(3,4,5,6-tetrahydrophthalimido) butyric acid.

**6.** Process according to any one of claims 4 and 5, wherein the imido-carboxylic acid substrate is gradually reacted in concentrated $H_2SO_4$ or $CH_3SO_3H$ with $H_2O_2$ having a concentration within the range of from about 70% to about 90% by weight, at a temperature of from 0 to about 15°C.

**7.** Process according to any one of claims 4 to 6, wherein $H_2SO_4$ or $CH_3SO_3H$ are employed in an amount of at least about 3 moles per mole of substrate.

**8.** Process according to claim 7, wherein $H_2SO_4$ or $CH_3SO_3H$ are employed in an amount of from about 3 to about 20 moles per mole of substrate.

**9.** Process according to any one of claims 4 to 8, wherein the hydrogen peroxide is employed in an amount of at least about 1.2 mole per mole of substrate.

**10.** Process according to claim 9, wherein the hydrogen peroxide is employed in an amount of from about 1.5 to about 6 moles per mole of substrate.

**11.** Process according to any one of claims 4 to 10, wherein the final molar ratio of $H_2SO_4$ or $CH_3SO_3H$ with respect to the total $H_2O$ present at the end of the reaction is at least about 1.0.

**12.** Process according to claim 11, wherein the final molar ratio of $H_2SO_4$ or $CH_3SO_3H$ with respect to the total $H_2O$ present at the end of the reaction is from about 1.2 to about 5.

**13.** Use of the imido-percarboxylic acids of formula (1) as defined in claim 1 as bleaching agents in detergent formulations optionally containing other components and/or additives.

**14.** Use according to claim 13, wherein the detergent formulations optionally contain builders, surfactants, soaps, zeolites, hydrotropic agents, corrosion inhibitors, enzymes, optical bleaching agents, stabilizers and other peroxy compounds.

**15.** Compound selected from 4-succinimido-perbutyric acid, 3-succinimido-perpropionic acid, succinimido-peracetic acid, 4-hexahydro-phthalimido-perbutyric acid, 3-hexahydro-phthalimido-perpropionic acid. 2-hexahydrophthalimido-3-methyl-perbutyric acid, 2-hexahydro-phthalimidoperpropionic acid or 4-(3,4,5,6-tetrahydro-phthalimido) perbutyric acid.

**16.** Process for preparing the compounds according to claim 15, wherein a substrate, the imido-carboxylic acid corresponding to the desired imido-percarboxylic acid of formula (I), is reacted with concentrated $H_2O_2$ by operating in a medium selected from concentrated $H_2SO_4$ and $CH_3SO_3H$, the resulting compound of formula (I) being separated thereafter from the reaction mixture by known techniques, wherein the substrate is selected from 4-succinimido butyric acid, 3-succinimido pro- pionic acid, succinimido acetic acid, 4-hexahydro-phthalimido butyric acid, 3-hexahydro-phthalimido propionic acid, 2-hexahydro-phthalimido-3-methyl-butyric acid, 2-hexahydrophthalimido propionic acid and 4-(3,4,5,6-tetrahydrophthalimido) butyric acid.

**Patentansprüche**

**1.** Imidopercarbonsäuren mit der allgemeinen Formel (I):

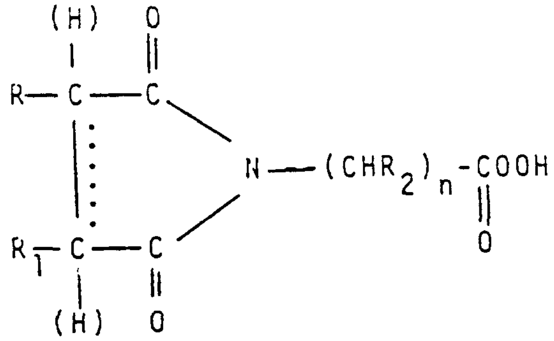

(I)

wobei:

R und $R_1$ gleich oder voneinander verschieden Wasserstoffatome oder lineare oder verzweigte $C_{1-12}$-Alkylgruppen darstellen oder R und $R_1$ zusammen mit den Kohlenstoffatomen, an denen sie gebunden sind, einen cycloaliphatischen Ring bilden, welcher von 4 bis 12 Kohlenstoffatome enthält; wobei das

Symbol ..... eine Einzelbindung oder eine olefinisch ungesättigte Bindung anzeigt;

$R_2$ ein Wasserstoffatom, eine lineare oder verzweigte $C_{1-5}$-Alkylgruppe oder eine OH-Gruppe darstellt; und n eine ganze Zahl von 1 bis 10 ist; wobei die durch R ,$R_1$ und $(CHR_2)_n$ dargestellten Gruppen gegebenenfalls mit einem oder mehreren aus F, Cl, OH, $NO_2$ und $C_1$-$C_5$-Alkoxygruppen ausgewählten Substituenten substituiert sind, wobei die Substituenten gleich oder voneinander verschieden sein können,

wobei die in den allgemeinen Formeln a), b) und c) definierten Verbindungen ausgenommen sind:

a) 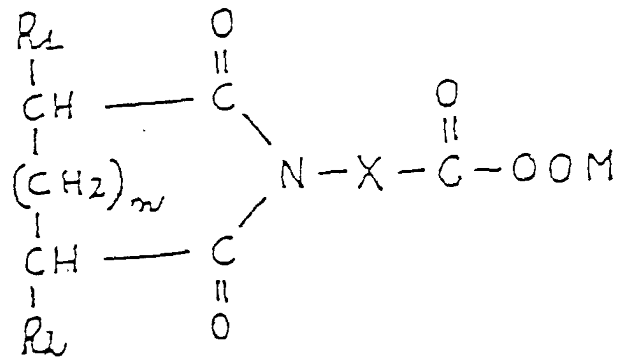

wobei gilt:

n = Null
$R_1$ = H, $C_1$-$C_{12}$-Alkyl
$R_2$ = H
M = H
X = $C_1$-$C_{10}$-Alkylen

b) 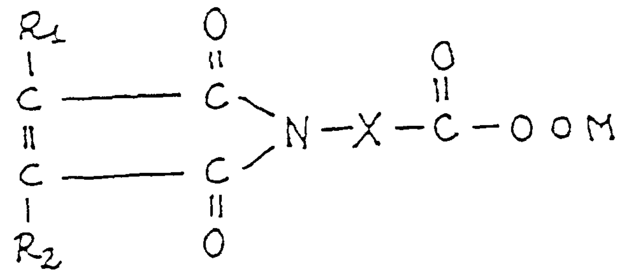

wobei gilt:

$R_1$ = H, $C_1$-$C_{12}$-Alkyl
$R_2$ = H
M = H
X = $C_1$-$C_{12}$-Alkylen

c) 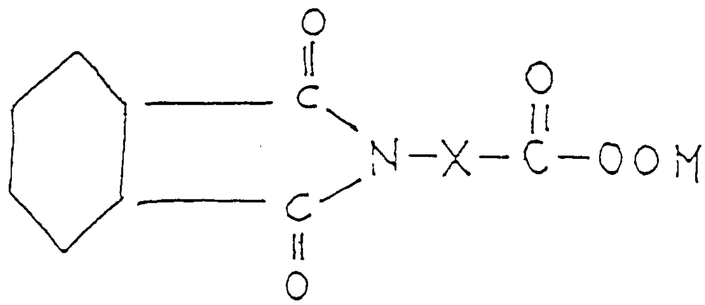

wobei gilt:

M = H
X = $C_1$-$C_{10}$-Alkylen.

**2.** Verbindungen gemäß Anspruch 1, wobei R und $R_1$ lineare oder verzweigte $C_{1-6}$-Alkylgruppen darstellen oder R und $R_1$ zusammen mit den Kohlenstoffatomen, an denen sie gebunden sind, einen cycloaliphatischen Ring bilden, welcher 5 oder 6 Kohlenstoffatome enthält, wobei die durch R und $R_1$ dargestellten Gruppen gegebenenfalls wie in Anspruch 1 angegeben substituiert sein können.

**3.** Verbindung gemäß Anspruch 1, nämlich 2-Hexahydrophthalimido-3-methylperbutansäure, 2-Hexahydrophthalimidoperpropionsäure oder 4-(3,4,5,6-Tetrahydrophthalimido)perbutansäure.

**4.** Verfahren zur Herstellung der Verbindungen gemäß Anspruch 1, wobei ein Substrat, die der gewünschten Imidopercarbonsäure der Formel (I) entsprechende Imidocarbonsäure, mit konzentriertem $H_2O_2$ umgesetzt wird, indem in einem aus konzentrierter $H_2SO_4$ und $CH_3SO_3H$ ausgewählten Medium gearbeitet wird, wobei anschließend die resultierende Verbindung der Formel (I) durch bekannte Techniken von der Reaktionsmischung abgetrennt wird.

**5.** Verfahren gemäß Anspruch 4, wobei das Substrat ausgewählt ist aus 2-Hexahydrophthalimido-3-methylbutansäure, 2-Hexahydrophthalimidopropionsäure oder 4-(3,4,5,6-Tetrahydrophthalimido)butansäure.

**6.** Verfahren gemäß einem der Ansprüche 4 und 5, wobei das Imidocarbonsäuresubstrat schrittweise in konzentrierter $H_2SO_4$ oder $CH_3SO_3H$ mit $H_2O_2$ mit einer Konzentration innerhalb des Bereichs von ungefähr 70 bis ungefähr 90 Gew.-% bei einer Temperatur von 0 bis ungefähr 15°C umgesetzt wird.

**7.** Verfahren gemäß einem der Ansprüche 4 bis 6, wobei $H_2SO_4$ oder $CH_3SO_3H$ in einer Menge von mindestens ungefähr 3 Mol pro Mol an Substrat verwendet werden.

**8.** Verfahren gemäß Anspruch 7, wobei $H_2SO_4$ oder $CH_3SO_3H$ in einer Menge von ungefähr 3 bis ungefähr 20 Mol pro Mol an Substrat verwendet werden.

**9.** Verfahren gemäß einem der Ansprüche 4 bis 8, wobei das Wasserstoffperoxid in einer Menge von mindestens ungefähr 1,2 Mol pro Mol an Substrat verwendet wird.

**10.** Verfahren gemäß Anspruch 9, wobei das Wasserstoffperoxid in einer Menge von ungefähr 1,5 bis ungefähr 6 Mol pro Mol an Substrat verwendet wird.

**11.** Verfahren gemäß einem der Ansprüche 4 bis 10, wobei das Endmolverhältnis von $H_2SO_4$ oder $CH_3SO_3H$ im Bezug auf das gesamte am Ende der Umsetzung vorhandene $H_2O$ mindestens ungefähr 1,0 beträgt.

**12.** Verfahren gemäß Anspruch 11, wobei das Endmolverhältnis von $H_2SO_4$ oder $CH_3SO_3H$ im Bezug auf das gesamte am Ende der Umsetzung vorhandene $H_2O$ von ungefähr 1,2 bis ungefähr 5 beträgt.

**13.** Verwendung der wie in Anspruch 1 definierten Imidopercarbonsäuren der Formel (I) als Bleichmittel in Waschmittelzubereitungen, welche gegebenenfalls andere Komponenten und/oder Additive enthalten.

**14.** Verwendung gemäß Anspruch 13, wobei die Waschmittelzubereitungen gegebenenfalls Gerüststoffe, Tenside, Seifen, Zeolithe, hydrotrope Mittel, Korrosionsinhibitoren, Enzyme, optische Bleichmittel, Stabilisatoren und andere Peroxyverbindungen enthalten.

**15.** Verbindung, ausgewählt aus 4-Succinimidoperbutansäure, 3-Succinimidoperpropionsäure, Succinimidoperessigsäure, 4-Hexahydrophthalimidoperbutansäure, 3-Hexahydrophthalimidoperpropionsäure, 2-Hexahydrophthalimido-3-methylperbutansäure, 2-Hexahydrophthalimidoperpropionsäure oder 4-(3,4,5,6-Tetrahydrophthalimido)perbutansäure.

**16.** Verfahren zur Herstellung der Verbindungen gemäß Anspruch 15, wobei ein Substrat, die der gewünschten Imidopercarbonsäure der Formel (I) entsprechende Imidocarbonsäure, mit konzentriertem $H_2O_2$ umgesetzt wird, indem in einem aus konzentrierter $H_2SO_4$ und $CH_3SO_3H$ ausgewählten Medium gearbeitet wird, wobei anschließend die resultierende Verbindung der Formel (I) durch bekannte Techniken von der Reaktionsmischung abgetrennt wird, und
wobei das Substrat ausgewählt ist aus 4-Succinimidobutansäure, 3-Succinimidopropionsäure, Succinimidoessigsäure, 4-Hexahydrophthalimidobutansäure, 3-Hexahydrophthalimidopropionsäure, 2-Hexahydrophthalimido-3-methylbutansäure, 2-Hexahydrophthalimidopropionsäure oder 4-(3,4,5,6-Tetrahydrophthalimido) butansäure.

## Revendications

**1.** Acides imido-percarboxyliques, représentés par la formule générale (I) :

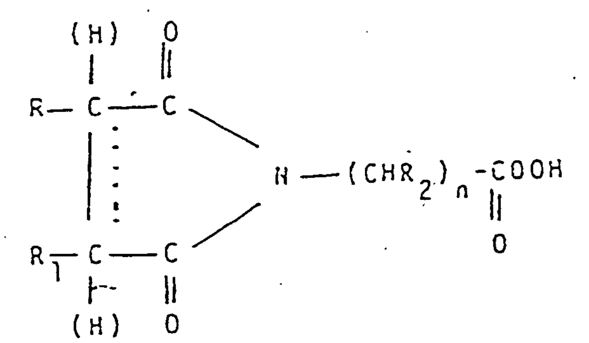

(I)

dans laquelle :

- R et $R_1$, identiques ou différents l'un de l'autre, représentent des atomes d'hydrogène ou des groupes alkyle en $C_{1-12}$, linéaires ou ramifiés, ou bien R et $R_1$, conjointement avec les atomes de carbone auxquels ils sont liés, forment un noyau cycloaliphatique contenant de 4 à 12 atomes de carbone ;
- le symbole ..... indique une simple liaison ou une liaison d'insaturation oléfinique ;
- $R_2$ représente un atome d'hydrogène, un groupe alkyle en $C_{1-5}$, linéaire ou ramifié, ou un groupe OH ; et
- n est un entier de 1 à 10 ;

les groupes représentés par R, $R_1$ et $(CHR_2)_n$ étant facultativement substitués par un ou plusieurs substituants choisis parmi F, Cl, OH, $NO_2$ et les groupes alcoxy en $C_{1-5}$, lesquels substituants peuvent être identiques ou différents les uns des autres,
à l'exclusion des composés définis par les formules générales a), b) et c) :

a)

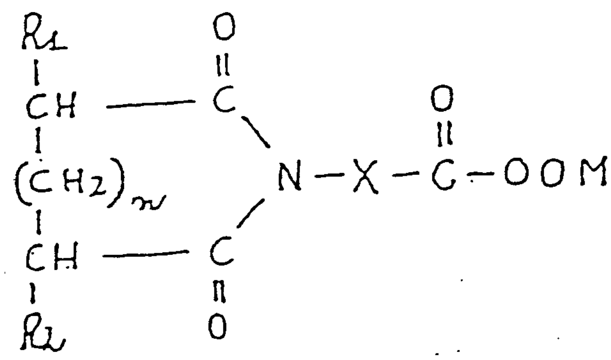

dans laquelle :

- n = zéro
- $R_1$ = H, alkyle en $C_1$-$C_{12}$
- $R_2$ = H
- M = H
- X = alkylène en $C_1$-$C_{10}$

b)

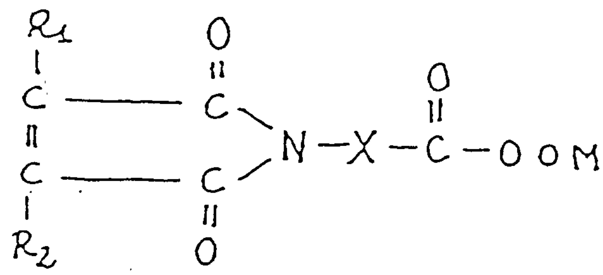

dans laquelle :

- $R_1$ = H, alkyle en $C_1$-$C_{12}$
- $R_2$ = H
- M = H
- X = alkylène en $C_1$-$C_{12}$

c)

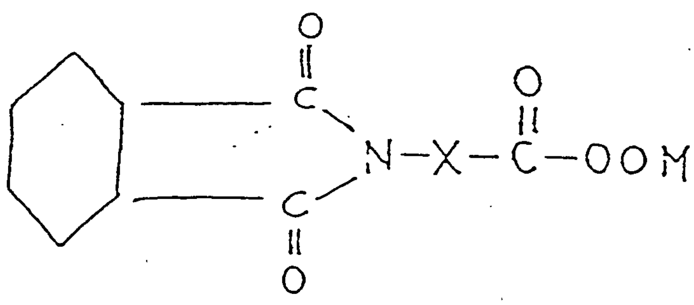

dans laquelle :

- M = H
- X = alkylène en $C_1$-$C_{10}$.

**2.** Composés selon la revendication 1, dans lesquels :

- R et $R_1$ représentent des groupes alkyle en $C_{1-6}$, linéaires ou ramifiés, ou bien R et $R_1$, conjointement avec les atomes de carbone auxquels ils sont liés, forment un noyau cycloaliphatique contenant 5 ou 6 atomes de carbone, lesquels groupes représentés par R et $R_1$ peuvent facultativement être substitués comme indiqué à la revendication 1.

**3.** Composé selon la revendication 1 qui est l'acide 2-hexahydro-phtalimido-3-méthyl-perbutyrique, l'acide 2-hexahydro-phtalimido-perpropionique ou l'acide 4-(3,4,5,6-tétrahydro-phtalimido)perbutyrique.

**4.** Procédé de préparation des composés tels que définis à la revendication 1, dans lequel un substrat, qui est l'acide imido-carboxylique correspondant à l'acide imido-percarboxylique désiré de formule (I), est mis à réagir avec $H_2O_2$ concentré en opérant dans un milieu choisi parmi $H_2SO_4$ et $CH_3SO_3H$ concentrés, le composé résultant de formule (I) étant séparé par la suite du mélange réactionnel par des techniques connues.

**5.** Procédé selon la revendication 4, dans lequel le substrat est choisi parmi l'acide 2-hexahydro-phtalimido-3-méthyl-butyrique, l'acide 2-hexahydrophtalimido propionique et l'acide 4-(3,4,5,6-tétrahydrophtalimido)butyrique.

**6.** Procédé selon l'une quelconque des revendications 4 et 5, dans lequel le substrat acide imido-carboxylique est mis à réagir progressivement dans $H_2SO_4$ ou $CH_3SO_3H$ concentré avec $H_2O_2$ ayant une concentration se situant à l'intérieur de la plage allant d'environ 70% à environ 90% en poids, à une température de 0 à environ 15°C.

**7.** Procédé selon l'une quelconque des revendications 4 à 6, dans lequel $H_2SO_4$ ou $CH_3SO_3H$ sont employés dans une quantité d'au moins environ 3 moles par mole de substrat.

**8.** Procédé selon la revendication 7, dans lequel $H_2SO_4$ ou $CH_3SO_3H$ sont employés dans une quantité allant d'environ 3 à environ 20 moles par mole de substrat.

**9.** Procédé selon l'une quelconque des revendications 4 à 8, dans lequel le peroxyde d'hydrogène est employé dans une quantité d'au moins environ 1,2 mole par mole de substrat.

**10.** Procédé selon la revendication 9, dans lequel le peroxyde d'hydrogène est employé dans une quantité allant d'environ 1,5 à environ 6 moles par mole de substrat.

**11.** Procédé selon l'une quelconque des revendications 4 à 10, dans lequel le rapport molaire final d'$H_2SO_4$ ou $CH_3SO_3H$ par rapport à l'$H_2O$ total présent à la fin de la réaction est d'au moins environ 1,0.

**12.** Procédé selon la revendication 11, dans lequel le rapport molaire final d'$H_2SO_4$ ou $CH_3SO_3H$ par rapport à l'$H_2O$ total présent à la fin de la réaction est d'environ 1,2 à environ 5.

**13.** Utilisation des acides imido-percarboxyliques de la formule (I) telle que définie à la revendication 1 en tant qu'agents de blanchiment dans des formulations détergentes contenant facultativement d'autre composants et/ou additifs.

**14.** Utilisation selon la revendication 13, dans laquelle les formulations détergentes contiennent facultativement des adjuvants pour détergents, des agents tensio-actifs, des savons, des zéolites, des agents hydrotropiques, des inhibiteurs de corrosion, des enzymes, des agents de blanchiment optique, des stabilisants et d'autres composés peroxy.

**15.** Composé choisi parmi l'acide 4-succinimidoperbutyrique, l'acide 3-succinimido-perpropionique, l'acide succinimido-peracétique, l'acide 4-hexahydro-phtalimidoperbutyrique, l'acide 3-hexahydro-phtalimido-perpropionique, l'acide 2-hexahydro-phtalimido-3-méthyl-perbutyrique, l'acide 2-hexahydro-phtalimido-perpropionique ou l'acide 4-(3,4,5,6-tétrahydro-phtalimido)perbutyrique.

**16.** Procédé de préparation des composés tels que définis à la revendication 15, dans lequel un substrat, qui est l'acide imido-carboxylique correspondant à l'acide imido-percarboxylique désiré de formule (I), est mis à réagir avec $H_2O_2$ concentré en opérant dans un milieu choisi parmi $H_2SO_4$ et $CH_3SO_3H$ concentrés, le composé résultant de formule (I) étant séparé par la suite du mélange réactionnel par des techniques connues, le substrat étant choisi parmi l'acide 4-succinimidobutyrique, l'acide 3-succinimidopropionique, l'acide succinimido acétique, l'acide

4-hexahydro-phtalimidobutyrique, l'acide 3-hexahydro-phtalimido-propionique, l'acide 2-hexahydro-phtalimido-3-méthyl-butyrique, l'acide 2-hexahydro-phtalimido propionique et l'acide 4-(3,4,5,6-tétrahydro-phtalimido)butyrique.